# EUROPÄISCHE PATENTSCHRIFT

(11) **EP 2 842 506 B1**
(45) Veröffentlichungstag und Bekanntmachung des Hinweises auf die Patenterteilung: **02.10.2019**
(21) Anmeldenummer: 14181270.1
(22) Anmeldetag: 18.08.2014
(51) Int. Cl.: A61B 18/14

(54) **Medizinisches Instrument und elektrochirurgisches System**
Medical instrument and electro-surgical system
Instrument médical et système électrochirurgical

(30) Priorität: 30.08.2013 DE 102013109505
(43) Veröffentlichungstag der Anmeldung: 04.03.2015
(73) Patentinhaber: Karl Storz SE & Co. KG, 78532 Tuttlingen (DE)
(72) Erfinder: Doll, Frank, 78607 Talheim (DE); Hahn, Martin, 88637 Leibertingen-Altheim (DE); Wittke, Uwe, 78532 Tuttlingen-Möhringen (DE)
(74) Vertreter: Witte, Weller & Partner Patentanwälte mbB

(56) Entgegenhaltungen:
- DE-A1- 4 032 601
- US-A1- 2005 251 134
- US-B1- 6 730 081

## Beschreibung

Bipolares medizinisches Instrument zum Schneiden von Gewebe unter der Wirkung von Hochfrequenzstrom, wobei sich das Instrument entlang einer Längsrichtung von einem proximalen Ende zu einem distalen Ende erstreckt und wobei am distalen Ende eine Aktivelektrode und eine der Aktivelektrode benachbarten Neutralelektrode angeordnet sind.

Die Erfindung betrifft ferner ein elektrochirurgisches System, mit einem Hochfrequenzgenerator und einem Instrument der eingangs genannten Art.

Ein Instrument und ein System der eingangs genannten Art sind aus DE 25 21 719 bekannt.

Ein Instrument bzw. ein System der eingangs genannten Art wird in der offenen, vorzugsweise aber in der minimal-invasiven Chirurgie zum Schneiden von Gewebe im menschlichen oder tierischen Körper verwendet.

Das eingangs genannte Instrument kann im Sinne der vorliegenden Erfindung bei Verwendung in der minimalinvasiven Chirurgie zusammen mit einem Endoskop zu einem sogenannten Resektoskop kombiniert werden oder selbst ein solches Resektoskop darstellen.

Die Elektro- oder Hochfrequenzchirurgie wird therapeutisch in verschiedenen medizinischen Fachdisziplinen wie bspw. der Urologie, Gynäkologie, Neurochirurgie, Abdominalchirurgie usw. eingesetzt. Speziell in der Urologie wird mittels eines eingangs genannten Instruments im Rahmen der minimal-invasiven Chirurgie endoskopisch Prostatagewebe abgetragen.

Bei elektrochirurgischen Verfahren der Resektoskopie unterscheidet man zwischen der monopolaren und der bipolaren Applikation des Hochfrequenzstromes.

Bei der monopolaren Applikation wird nur die Aktivelektrode, manchmal auch als Schneidelektrode oder Behandlungselektrode bezeichnet, in das Behandlungsgebiet eingeführt, während die Neutralelektrode außen am Patienten angeordnet wird. Folglich führt der Stromfluss zwischen der Aktivelektrode und der Neutralelektrode durch den Körper des Patienten hindurch, wobei der Nachteil besteht, dass der Strompfad durch den Patienten nicht sicher kontrolliert werden kann mit der Folge möglicher Verletzungen von Organen. Darüber hinaus kann die am Patientenkörper angebrachte Neutralelektrode zu Verbrennungen der Haut des Patienten führen.

Bei der bipolaren Technik, von der die vorliegende Erfindung ausgeht, werden sowohl Aktivelektrode als auch Neutralelektrode in das Behandlungsgebiet eingeführt. Der Stromfluss kann dadurch in kontrollierbarer Weise auf den Bereich zwischen der Aktivelektrode und der Neutralelektrode begrenzt werden, dass er räumlich nur zwischen der Aktivelektrode und der Neutralelektrode fließt. Entsprechend sind medizinisehe Instrumente der eingangs genannten Art geschaffen worden, bei denen die Aktivelektrode und die Neutralelektrode an einem Elektrodenträger angeordnet sind, so dass die Aktivelektrode und die Neutralelektrode einander benachbart in das Behandlungsgebiet eingebracht werden können.

Bei bipolaren medizinischen Instrumenten der eingangs genannten Art ist die Aktivelektrode üblicherweise mit einer kleinen wirksamen Oberfläche ausgebildet, so dass sich an der Aktivelektrode eine hohe Stromdichte einstellt, während die Neutralelektrode üblicherweise relativ großflächig ausgebildet ist, so dass sich an der Neutralelektrode nur mittlere oder geringe Stromdichten einstellen. Mit der Aktivelektrode wird entsprechend geschnitten, während die Neutralelektrode möglichst keine Wirkung auf das Gewebe ausüben soll, sondern lediglich der Begrenzung des Strompfades auf den Bereich zwischen der Aktivelektrode und der Neutralelektrode dienen soll.

Bei dem aus der oben genannten DE 25 21 719 bekannten Instrument ist die Neutralelektrode als breites Band ausgebildet, während die Aktivelektrode als Drahtschlinge ausgebildet ist. In einem weiteren Ausführungsbeispiel der DE 25 21 719 ist die Neutralelektrode auf ihrer der Aktivelektrode abgewandten Seite von einem Kunststoff-Fortsatz, der fest mit dem Schaft des Resektoskops verbunden ist, überdeckt.

WO 99/16371 A1 offenbart ebenfalls ein bipolares medizinisches Instrument der eingangs genannten Art. Ähnlich zu dem zuvor beschriebenen bekannten Instrument ist die Aktivelektrode zur Neutralelektrode in Richtung quer zur Längsachse so positioniert, dass diese nicht voneinander beabstandet sind.

Aus EP 1 163 886 A2 ist ein resektoskopisches Instrument bekannt, bei dem die Aktivelektrode und die Neutralelektrode auf ihren einander zugewandten Seiten durch einen Isolatorkörper elektrisch getrennt sind, so dass jede gerade Verbindungslinie zwischen den Elektroden durch den Isolatorkörper verläuft. Dadurch soll der direkte Stromfluss zwischen den bei den Elektroden erschwert bzw. vermindert werden.

US 6,730,081 zeigt ein endoskopisches Instrument zum verändernden Bearbeiten von Gewebe, Es werden eine Vielzahl von Elektrodenformen aufgezeigt, die, wenn eine Aktivelektrode und eine Neutralelektrode verwendet werden, einen gleichförmig orientierten Verlauf haben.

US 2005/0251134 A1 zeigt ein endoskopisches Instrument zum verändernden Bearbeiten von Gewebe, wobei bei einigen Ausgestaltungen zwei bogenförmige Elektroden in Längsrichtung hintereinander angeordnet sind.

DE 40 32 601 A1 zeigt ein endoskopisches Instrument zum verändernden Bearbeiten von Gewebe mit einem isolierten Schlingenträger und einer nicht isolierten Schneidschlinge, wobei am Schlingenträger ein Distanzhalter vorgesehen ist, der sich distal vor die Schneidschlinge sowie nach unten erstreckt.

Die bekannten Instrumente sind entweder nicht für einen Einsatz in besonders kleinen Hohlräumen geeignet, sind in der Herstellung zu teuer oder bieten keinen ausreichenden Schutz, um ungewollte Perforationen in den Wänden der Hohlräume zu vermeiden.

Der Erfindung liegt die Aufgabe zu Grunde, ein verbessertes Instrument sowie ein verbessertes System der eingangs genannten Art bereitzustellen

Gemäß einem ersten Aspekt der Erfindung wird ein eingangs genanntes medizinisches Instrument aufgezeigt, bei dem die Neutralelektrode einen gekrümmten Verlauf mit einer ersten und einer zweiten Krümmung hat, wobei die jeweiligen Richtungen der Krümmungen unterschiedlich sind.

Eine solche Form kann atraumatisch wirken, da die Neutralelektrode an ihrer distalseitigen Begrenzung nicht mehr über eine geradlinige Kante verfügt, sondern am distalen Ende einen gekrümmten Verlauf hat. Während eine geradlinige Kante ein gewisses Risiko bietet, dass der Arzt bei dem Einsatz des Instruments mit der Neutralelektrode unerwünscht in Gewebe schneidet, wird dieses Risiko durch die vorgeschlagene Ausgestaltung reduziert.

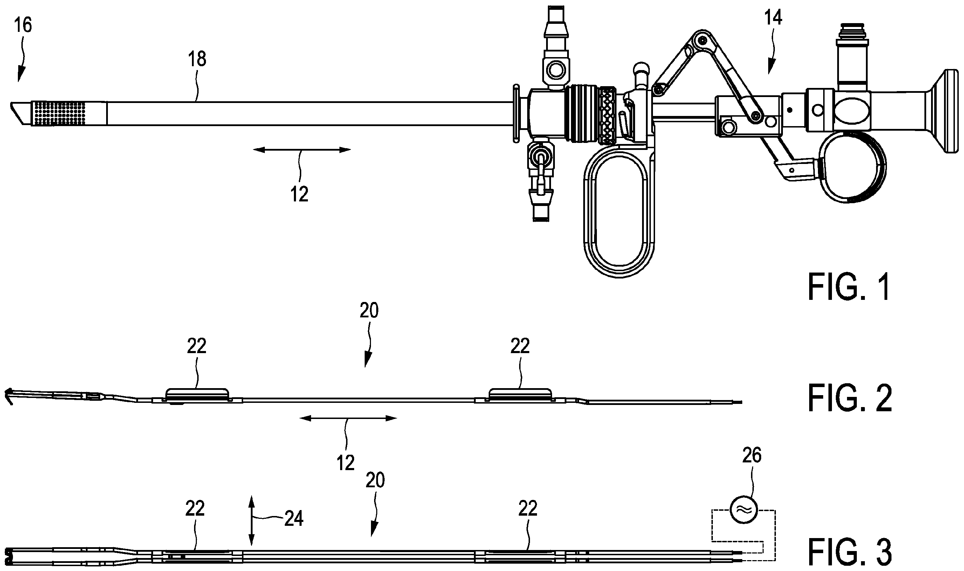

Für eine bessere Orientierung sollen die Krümmungen insbesondere in eine Projektionsebene betrachtet werden, die von der Längsrichtung und einer hierzu quer verlaufenden Querrichtung aufgespannt wird. Die Querrichtung soll dabei insbesondere so verstanden werden, dass sie die Richtung der geradlinigen Verbindungsstrecke zwischen den beiden Punkten hat, an denen die Neutralelektrode relativ zum Instrument gehalten ist. In der später folgenden Fig. 3 stellt die Zeichenebene diese genannte Projektionsebene dar.

Die Radien der Krümmungen sind bei einer bevorzugten Ausgestaltung gleich groß und bei einer anderen bevorzugten Ausgestaltung unterschiedlich groß gewählt. Bei bestimmten bevorzugten Ausführungsformen haben eine, mehrere oder alle Krümmungen einen konstanten Radius, während bei anderen bevorzugten Ausführungsformen eine, mehrere oder alle Krümmungen einen variierenden Radius haben, wobei insbesondere eine Schneckenform gebildet sein kann. Es ist bevorzugt, wenn die Aktivelektrode als Drahtschlinge ausgeführt ist.

Dadurch ist die Aufgabe vollständig gelöst.

Bei einer bevorzugten Ausgestaltung hat der gekrümmte Verlauf mindestens drei Krümmungen und ändert sich entlang des Verlaufs der Neutralelektrode die Richtungen der Krümmungen zweimal.

Auf diese Weise kann die Neutralelektrode besonders atraumatisch ausgestaltet werden. Bei bestimmten Ausführungsformen weist die Neutralelektrode vier oder mehr Krümmungen auf. Es obliegt dem Fachmann im Hinblick auf die jeweilige Anwendung und die maximal gewünschte Größe der Neutralelektrode, hier die geeignete Wahl zu treffen. Soll die Neutralelektrode besonders klein ausgeführt sein, wie dies insbesondere bei urologischen Maßnahmen gewünscht ist, wird eine Ausgestaltung mit drei Krümmungen als besonders vorteilhaft angesehen. Bei weiteren bevorzugten Ausgestaltungen hat der gekrümmte Verlauf mindestens vier Krümmungen, mindestens fünf Krümmungen oder mindestens sechs Krümmungen.

Bei einer weiteren vorteilhaften Ausgestaltung ist die Neutralelektrode als Drahtelement ausgebildet.

Diese Ausgestaltung kann fertigungstechnisch besonders einfach realisiert werden. Der Draht kann einen Umfang haben, der sich aus geraden Linien und/oder Bögen zusammensetzt.

Bei einer weiteren vorteilhaften Ausgestaltung ist eine erste Oberfläche der Aktivelektrode kleiner als eine zweite Oberfläche der Neutralelektrode.

Diese Ausgestaltung ermöglicht es, dass bei einer Gewebeberührung ein Zünden möglichst an der Aktivelektrode stattfindet und nicht an der Neutralelektrode, selbst wenn diese unerwünscht Gewebe berührt. Dabei ist es bevorzugt, die größere zweite Oberfläche dadurch zu erzielen, dass zumindest ein mittlerer Querschnitt der Neutralelektrode größer ist als ein mittlerer Querschnitt der Aktivelektrode und/oder dass die Erstreckung der Neutralelektrode entlang ihres physikalischen Verlaufs größer ist als die Erstreckung der Aktivelektrode.

Bei einer bevorzugten Ausgestaltung ist die Erstreckung der Neutralelektrode entlang ihres physikalischen Verlaufs im Vergleich zu der Erstreckung der Aktivelektrode entlang ihres physikalischen Verlaufs um mindestens 5 % größer, bevorzugt um mindestens 10 % größer, besonders bevorzugt um mindestens 20 % größer und insbesondere um mindestens 30 % größer.

Bei einer weiteren bevorzugten Ausgestaltung der Erfindung hat ein Querschnittsprofil der Neutralelektrode eine gerundete Form, insbesondere eine zumindest im Wesentlichen ovale oder zumindest im Wesentlichen kreisrunde Form.

Diese Ausgestaltung wird fertigungstechnisch als besonders vorteilhaft angesehen.

Bei einer weiteren bevorzugten Ausgestaltung der Erfindung weist die Neutralelektrode entlang ihres physikalischen Verlaufs einen gleichbleibenden Querschnitt auf, insbesondere einen konstanten Durchmesser.

Auch diese Ausgestaltung wird im Hinblick auf die Herstellung als vorteilhaft angesehen.

Bei einer weiteren vorteilhaften Ausgestaltung der Erfindung weist die Neutralelektrode in der Draufsicht zumindest in etwa eine Wellenform auf, insbesondere zumindest in etwa eine W-Form.

Eine solche Ausgestaltung wird besonders vorteilhaft im Hinblick auf eine atraumatische Anwendung angesehen. In diesem Zusammenhang ist es bevorzugt, wenn die W-Form großzügig gerundet ist. Großzügig im Rahmen dieser Anmeldung soll dabei so verstanden werden, dass der Radius einer Krümmung mindestens 4 %, bevorzugt mindestens 8 %, besonders bevorzugt mindestens 11 % und insbesondere mindestens 13 % der Erstreckung der Neutralelektrode in Querrichtung beträgt.

Bei einer weiteren vorteilhaften Ausgestaltung der Erfindung weist die Neutralelektrode zumindest zwei gebogene Abschnitte auf, insbesondere zumindest drei gebogene Abschnitte, die insbesondere jeweils zumindest in etwa die Form eines Teilovals oder eines Teilkreises haben.

In einer solchen Ausgestaltung wird eine gute atraumatische Wirkung gesehen, insbesondere, wenn die Radien der gebogenen Abschnitte großzügig bemessen sind.

Bei einer weiteren vorteilhaften Ausgestaltung befinden sich die Aktivelektrode und die Neutralelektrode in einem eingefahrenen Zustand innerhalb eines distalen Endes eines Schafts des Instruments.

Diese Ausgestaltung ermöglicht es, das Instrument besonders atraumatisch in den zu behandelnden Hohlraum einzuführen.

Bei einer weiteren bevorzugten Ausgestaltung ist, bezogen auf die Längsrichtung, ein distales Ende der Aktivelektrode weiter distal angeordnet als ein distales Ende der Neutralelektrode oder ist, bezogen auf die Längsrichtung, ein distales Ende der Aktivelektrode distal gleich weit angeordnet wie ein distales Ende der Neutralelektrode.

Diese Ausgestaltung hilft dabei sicherzustellen, dass die Aktivelektrode zuerst mit Gewebe in Kontakt tritt oder zumindest zeitgleich mit der Neutralelektrode mit Gewebe in Kontakt tritt. Dies wiederum unterstützt, dass das Zünden an der Aktivelektrode stattfindet und nicht an der Neutralelektrode. Unter dem Begriff des Zündens wird verstanden, dass sich an einer Elektrode, hier der Aktivelektrode, ein Plasma ausbildet, das Gewebe verdampft bzw. schneidet.

Bei einer weiteren vorteilhaften Ausgestaltung der Erfindung ist die Neutralelektrode zusammen mit dem Neutralleiter einstückig aus genau einem Material oder genau einer Materialmischung gefertigt.

Diese Ausgestaltung wird fertigungstechnisch als besonders günstig angesehen. Dabei sind die Neutralelektrode und der Neutralleiter bevorzugt aus einem zusammenhängenden Material gebildet und nicht durch formverbindende Maßnahmen, wie z.B. durch Pressen, Löten, Schweißen, Ultraschallbehandlung etc., verbunden sind. Als Neutralleiter soll hier der Leiter verstanden werden, der das eine für den Hochfrequenzstrom benötigte Potential vom proximalen Ende des Instruments bis zur Neutralelektrode am distalen Ende des Instruments führt.

Bei einer weiteren vorteilhaften Ausgestaltung der Erfindung erstreckt sich die Neutralelektrode bezogen auf ihre Quererstreckung in einem mittleren Bereich weniger weit distal als in Seitenbereichen der Neutralelektrode.

Diese Ausgestaltung ermöglicht es, ein Sichtfeld einer optischen Einrichtung am distalen Ende des Instruments zu verbessern. Während es im Stand der Technik häufig so ist, dass die Neutralelektrode einen nennenswerten Teil des Sichtfelds verdeckt, erlaubt es diese Ausgestaltung durch eine gezielte bereichsweise Verkleinerung der Neutralelektrode, ein vergrößertes Sichtfeld zu erhalten. Ferner kann diese Ausgestaltung auch einer nochmals verbesserten atraumatischen Handhabung dienen.

Bei einer weiteren vorteilhaften Erfindung verläuft zumindest ein Abschnitt der Neutralelektrode in einer Ebene, die in einem Winkel zur Längserstreckung des Instruments steht.

Auch diese Ausgestaltung ermöglicht ein vergrößertes Sichtfeld. Dabei ist es insbesondere bevorzugt, wenn zumindest eine Krümmung in dieser Ebene verläuft.

Bei einer weiteren vorteilhaften Ausgestaltung der Erfindung ist eine distalseitige Begrenzung der Neutralelektrode so geformt, dass zwischen einem ersten und einem zweiten Bereich der Neutralelektrode, die sich distal am weitesten erstrecken, ein dritter Bereich angeordnet ist, der sich distal weniger erstreckt als der erste und der zweite Bereich.

Diese Ausgestaltung dient ebenfalls einer atraumatischen Handhabung des Instruments.

Gemäß einem zweiten Aspekt wird ein elektrochirurgisches System mit einem Hochfrequenzgenerator und einem zuvor beschriebenen Instrument bereitgestellt, wobei das Instrument an den Hochfrequenzgenerator anschließbar ist und der Hochfrequenzgenerator insbesondere in der Lage ist, eine Ausgangsleistung von mindestens 50 W abzugeben, bevorzugt mindestens 100 W, besonders bevorzugt mindestens 150 W und insbesondere mindestens 200W. Bei bestimmten Ausführungsformen des elektrochirurgischen Systems ist der Hochfrequenzgenerator in der Lage ist, eine Ausgangsleistung von mindestens 350 W abzugeben.

Weitere Vorteile und Merkmale ergeben sich aus der nachfolgenden Beschreibung und der beigefügten Zeichnung.

Ausführungsbeispiele der Erfindung sind in der Zeichnung näher dargestellt und werden in der nachfolgenden Beschreibung näher erläutert. Es zeigen:
- Fig. 1: ein bipolares medizinisches Instrument gemäß der vorliegenden Erfindung in der Seitenansicht, hier ein Resektoskop;
- Fig. 2: Elemente innerhalb des Instruments gemäß Fig. 1 in der Seitenansicht;
- Fig. 3: Elemente innerhalb des Instruments gemäß Fig. 1 in der Draufsicht;
- Fig. 4: die Elemente gemäß Fig. 2 in perspektivischer Ansicht;
- Fig. 5: den vergrößerten Ausschnitt A aus Fig. 4;
- Fig. 6: eine etwas vergrößerte Darstellung der Fig. 2;
- Fig. 7: den vergrößerten Ausschnitt B aus Fig. 6;
- Fig. 8: eine etwas vergrößerte Darstellung der Fig. 3;
- Fig. 9: den vergrößerten Ausschnitt C aus Fig. 8;
- Fig. 10: zeigt die Neutralelektrode gemäß der zuvor beschriebenen ersten Ausführungsform;
- Fig. 11: zeigt eine Neutralelektrode gemäß einer zweiten Ausführungsform;
- Fig. 12: zeigt eine Neutralelektrode gemäß einer dritten Ausführungsform;
- Fig. 13: zeigt eine Neutralelektrode gemäß einer vierten Ausführungsform;
- Fig. 14: zeigt eine Neutralelektrode gemäß einer fünften Ausführungsform;
- Fig. 15: zeigt eine Neutralelektrode gemäß einer sechsten Ausführungsform;
- Fig. 16: zeigt eine Neutralelektrode gemäß einer siebten Ausführungsform;
- Fig. 17: zeigt eine Neutralelektrode gemäß einer achten Ausführungsform; und
- Fig. 18: zeigt eine Neutralelektrode gemäß einer neunten Ausführungsform.

Fig. 1 zeigt ein bipolares medizinisches Instrument 10 zum Schneiden von Gewebe unter der Wirkung von Hochfrequenzstrom in der Seitenansicht, hier ein Resektoskop. Die grundlegende Funktionsweise des Instruments 10 ist aus dem Stand der Technik bekannt, siehe hierzu insbesondere die Patentschrift EP 1 567 079 B1, Figuren 1-4, 6a und 6b, sowie Absätze 47 bis 72.

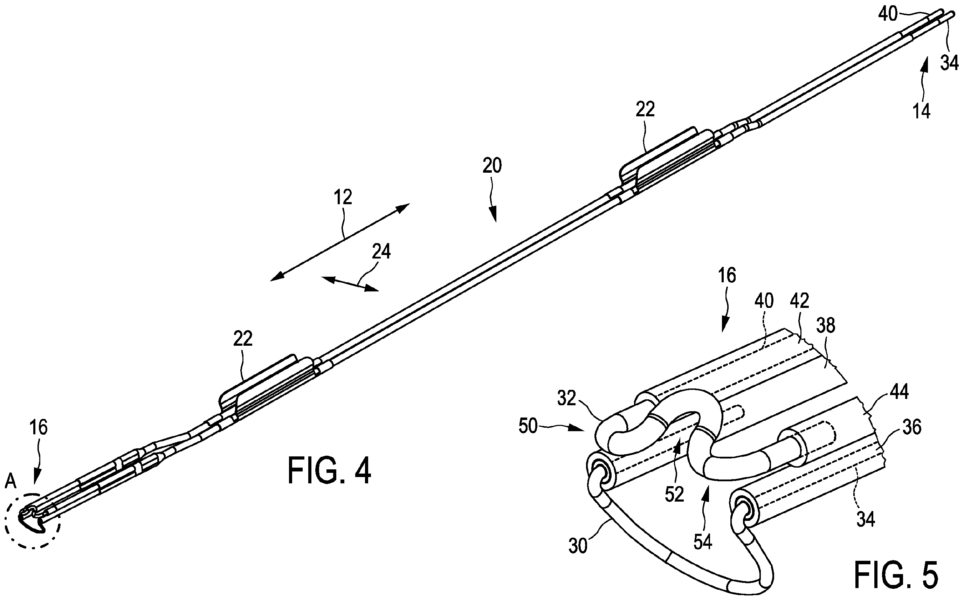

Das Instrument 10 erstreckt sich entlang einer Längsrichtung 12 von einem proximalen Ende 14 zu einem distalen Ende 16. Das Instrument 10 ist hier im eingefahrenen Zustand gezeigt. Es ist zu erkennen, dass sowohl die Aktivelektrode 30 (siehe Fig. 5) als auch die Neutralelektrode 32 (siehe Fig. 5) sich innerhalb eines distalen Endes eines Schafts 18 des Instruments 10 befinden.

Fig. 2 zeigt in der Seitenansicht einen Elektrodenträger 20, der innerhalb des Schafts 18 in Längsrichtung 12 verlagerbar angeordnet ist und von Halteelementen 22 geführt wird.

Fig. 3 zeigt den Elektrodenträger 20 und die Halteelemente 22 in der Draufsicht. Des Weiteren ist eine Querrichtung 24 eingezeichnet. Ferner ist symbolisch dargestellt, wie die elektrisch aktiven Elemente des Instruments 10 an einen Hochfrequenzgenerator 26 angeschlossen werden können, wobei der Hochfrequenzgenerator 26 hier eine Ausgangsleistung von mindestens 200 W abgeben kann.

Fig. 4 zeigt die Elemente gemäß Fig. 2 in perspektivischer Ansicht. Am distalen Ende 16 ist ein Teil der Figur mit einem Kreis und dem Buchstaben A gekennzeichnet, der nachfolgend vergrößert dargestellt wird.

Fig. 5 zeigt vergrößert den Ausschnitt A aus Fig. 4. Es ist zu erkennen, dass am distalen Ende 16 eine Aktivelektrode 30 und eine der Aktivelektrode 30 benachbarte Neutralelektrode 32 angeordnet sind. Die Aktivelektrode 30 ist als Drahtschlinge ausgeführt.

Der Aktivleiter 34, hier mit gestrichelten Linien dargestellt, der die Aktivelektrode 30 elektrisch mit dem Hochfrequenzgenerator 26 verbindet, ist in einem isolierenden Rohr 36 gehalten. Auf der anderen Seite ist ein mit der Aktivelektrode 30 verbundener Leiter in einem isolierenden Rohr 38 gehalten. Der Leiter kann zwar wie der Aktivleiter 34 ebenfalls zum selben Pol des Hochfrequenzgenerators 26 geführt sein, doch ist es ausreichend, wenn der Leiter innerhalb des Rohrs 38 endet, wie es hier anhand der gestrichelten Linie angedeutet ist. Der Leiter gibt der Aktivelektrode 30 also lediglich mechanischen Halt.

Der Neutraleiter 40, hier mit gestrichelten Linien dargestellt, der die Neutralelektrode 32 elektrisch mit dem Hochfrequenzgenerator 26 verbindet, ist in einem isolierenden Rohr 42 gehalten. Auf der anderen Seite ist ein mit der Neutralelektrode 32 verbundener Leiter in einem isolierenden Rohr 44 gehalten. Der Leiter kann zwar wie der Neutralleiter 40 ebenfalls zum selben Pol des Hochfrequenzgenerators 26 geführt sein, doch ist es ausreichend, wenn der Leiter innerhalb des Rohrs 44 endet, wie es hier anhand der gestrichelten Linie angedeutet ist. Der Leiter gibt der Aktivelektrode 30 also lediglich mechanischen Halt.

Es ist zu erkennen, dass die Neutralelektrode 32 einen gekrümmten Verlauf mit einer ersten und einer zweiten Krümmung 50, 52 hat, wobei die jeweiligen Richtungen der Krümmungen 50, 52 unterschiedlich sind.

Bei der hier gezeigten Ausführungsform weist der gekrümmte Verlauf zudem eine dritte Krümmung 54 auf, und die Richtungen der Krümmungen ändern sich zweimal entlang des Verlaufs der Neutralelektrode 32. Die Neutralelektrode 32 ist hier als Drahtelement ausgebildet. Eine erste Oberfläche der Aktivelektrode 30 ist kleiner als eine zweite Oberfläche der Neutralelektrode 32.

Dies wird bei dieser Ausführungsform insbesondere dadurch erreicht, dass der mittlere Querschnitt der Neutralelektrode größer ist, insbesondere deutlich größer ist, als der mittlere Querschnitt der Aktivelektrode.

Ein Querschnittsprofil der Neutralelektrode 32 hat hier eine gerundete Form, die hier zumindest im Wesentlichen kreisrund ist. Im Hinblick auf den genannten größeren Querschnitt kann man hier daher auch davon sprechen, dass der mittlere Durchmesser der Neutralelektrode 32 größer, insbesondere deutlich größer ist als der mittlere Durchmesser der Aktivelektrode 30.

Bei der hier gezeigten Ausführungsform ist die Neutralelektrode 32 zusammen mit dem Neutralleiter 40 einstückig aus genau einem Material, oder genau einer Materialmischung, gefertigt. Ebenso ist hier die Aktivelektrode 30 zusammen mit dem Aktivleiter 34 einstückig aus einem genau einem Material oder genau einer Materialmischung gefertigt. Insbesondere sind Aktivelektrode 30 und Aktivleiter 34 und auch die Neutralelektrode 32 und der Neutralleiter 40 jeweils zusammen aus einem Draht gebildet, der vom proximalen Ende des Elektrodenträgers 20 bis zum distalen Ende 16 des Instruments 10 führt.

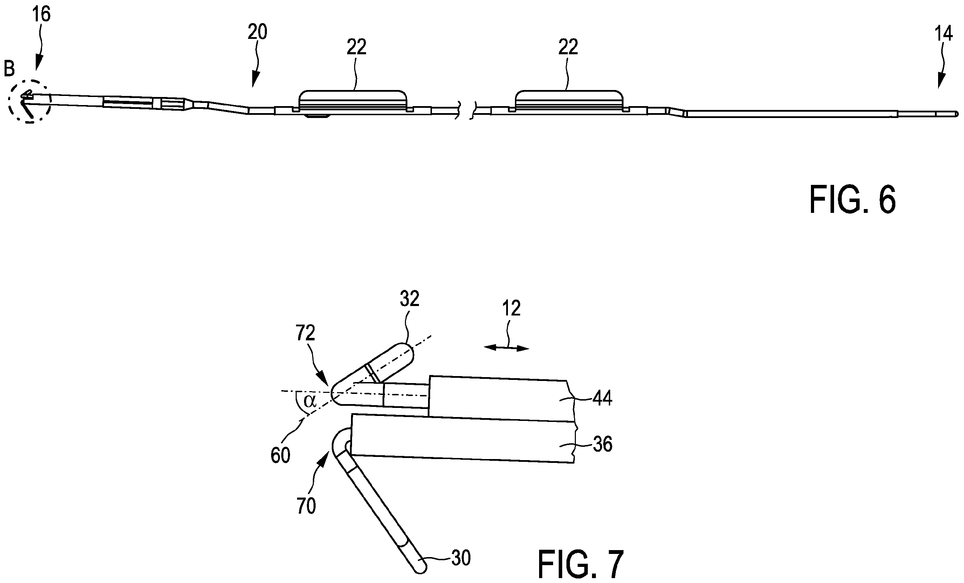

Fig. 6 zeigt eine etwas vergrößerte Darstellung der Fig. 2. Am distalen Ende 16 ist ein Teil der Figur mit einem Kreis und dem Buchstaben B gekennzeichnet, der nachfolgend vergrößert dargestellt wird.

Fig. 7 zeigt vergrößert den Ausschnitt B aus Fig. 6. Es ist zu erkennen, dass ein Abschnitt der Neutralelektrode 32, hier enthält der Abschnitt die zweite Krümmung 52, in einer Ebene 60 verläuft, die in einem Winkel α zu der Längsrichtung 12 des Instruments 10 steht. Die Ebene 60 steht hier senkrecht zur Zeichenebene und ist daher nur mit einer gestrichelten Linie angedeutet. bezogen auf die Längsrichtung 12 ist ein distales Ende 70 der Aktivelektrode 30 distal gleich weit angeordnet wie ein distales Ende 72 der Neutralelektrode 32. Bei einer anderen Ausführungsform (nicht gezeigt) ist das distale Ende 70 der Aktivelektrode 30 weiter distal angeordnet als ein distales Ende 72 der Neutralelektrode 32. Bezogen auf die Orientierung in der Zeichnung würde die Aktivelektrode 30 in diesem Fall weiter links angeordnet sein.

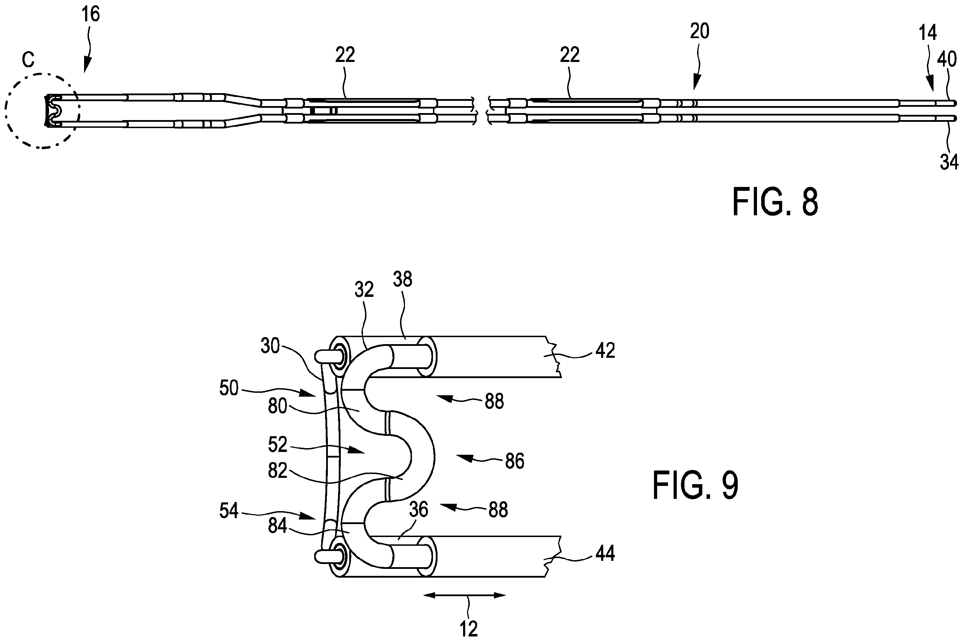

Fig. 8 zeigt eine etwas vergrößerte Darstellung der Fig. 3. Am distalen Ende 16 ist ein Teil der Figur mit einem Kreis und dem Buchstaben C gekennzeichnet, der nachfolgend vergrößert dargestellt wird.

Fig. 9 zeigt vergrößert den Ausschnitt C aus Fig. 8. Die Neutralelektrode 32 hat in der Draufsicht zumindest in etwa eine Wellenform, hier insbesondere zumindest in etwa eine W-Form. Die Neutralelektrode 32 weist zumindest zwei gebogene auf, hier drei gebogene Abschnitte 80, 82, 84, die hier jeweils zumindest in etwa die Form eines Halbkreises bzw. eines Halbtorus haben. Die Neutralelektrode 32 erstreckt sich, bezogen auf ihre Quererstreckung entlang der Querrichtung 24, in einem mittleren Bereich 86 weniger weit distal als in Seitenbereichen 88.

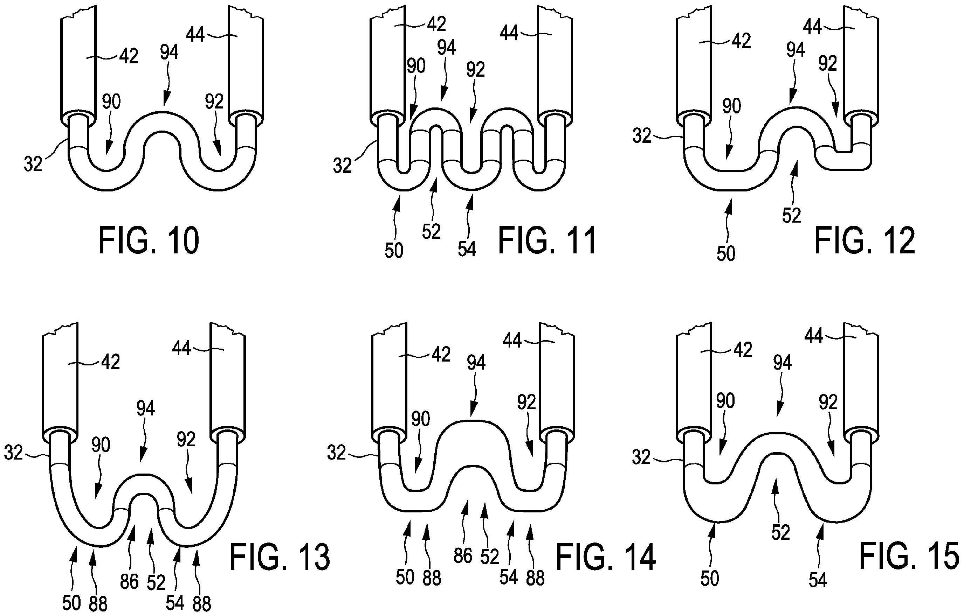

Fig. 10 zeigt nur die Neutralelektrode 32 in ihren isolierenden Rohren 42, 44 gemäß der zuvor beschriebenen ersten Ausführungsform. Eine distalseitige Begrenzung der Neutralelektrode 32, bezogen auf die Orientierung in der Zeichnung der untere geschwungene Verlauf der Neutralelektrode 32, ist hier so geformt, dass zwischen einem ersten und einem zweiten Bereich 90, 92 der Neutralelektrode 32, die sich distal am weitesten erstrecken, ein dritter Bereich 94 angeordnet ist, der sich distal weniger weit erstreckt als der erste und der zweite Bereich 90, 92.

Nachfolgend werden weitere Ausführungsformen der Neutralelektrode 32 aufgezeigt. Dabei werden aus Gründen der Übersichtlichkeit nicht mehr alle Bezugszeichen dargestellt, sondern lediglich einige ausgewählte Bezugszeichen, die eine Orientierung erleichtern. Grundsätzlich gelten für die nachfolgenden Ausführungsformen die Erläuterungen zur ersten Ausführungsform, sofern diese nicht offensichtlich unvereinbar mit der konkreten Ausführungsform sind.

Fig. 11 zeigt eine Neutralelektrode 32 gemäß einer zweiten Ausführungsform. Der gekrümmte Verlauf weist hier insgesamt fünf Krümmungen auf, deren Richtung sich viermal ändert.

Fig. 12 zeigt eine Neutralelektrode 32 gemäß einer dritten Ausführungsform. Die Ausführungsform zeigt, dass, auch wenn eine Symmetrie zur Mittelachse des Instruments 10 als vorteilhaft angesehen wird, eine solche Symmetrie nicht zwingend erforderlich ist. Insbesondere können die Anzahl der Krümmungen und die Radien der Krümmungen im Ermessen des Fachmanns gewählt werden.

Fig. 13 zeigt eine Neutralelektrode 32 gemäß einer vierten Ausführungsform. Es ist zu erkennen, dass nicht nur kreisrunde Krümmungen gewählt werden können, sondern auch Rundungen anderer Art. Die Radien der Krümmungen können grundsätzlich sehr klein gewählt werden, sofern das distale Ende der Neutralelektrode 32 keine Spitze ausbildet, die traumatisch wirken kann.

Fig. 14 zeigt eine Neutralelektrode 32 gemäß einer fünften Ausführungsform. Hier ist die zweite Krümmung 52 bzw. der dritte Bereich 94 mit einem vergrößerten Durchmesser ausgeführt.

Fig. 15 zeigt eine Neutralelektrode 32 gemäß einer sechsten Ausführungsform. Hier sind die erste und die dritte Krümmung 50, 54 bzw. der erste und der zweite Bereich 90, 92 mit einem vergrößerten Durchmesser ausgeführt.

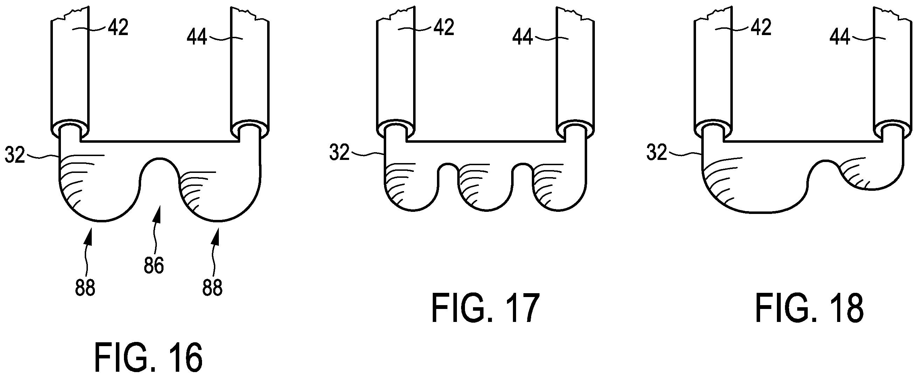

Fig. 16 zeigt eine Neutralelektrode 32 gemäß einer siebten Ausführungsform. Die siebte Ausführungsform entspricht an ihrer distalseitigen Begrenzung der ersten Ausführungsform, siehe Fig. 10. Es ist zu erkennen, dass die Neutralelektrode 32 nicht als Draht ausgeführt sein muss, sondern auch flächig ausgeführt sein kann.

Fig. 17 zeigt eine Neutralelektrode 32 gemäß einer achten Ausführungsform. Die achte Ausführungsform entspricht an ihrer distalseitigen Begrenzung der zweiten Ausführungsform, siehe Fig. 11. Es ist zu erkennen, dass die Neutralelektrode 32 nicht als Draht ausgeführt sein muss, sondern auch flächig ausgeführt sein kann.

Fig. 18 zeigt eine Neutralelektrode 32 gemäß einer neunten Ausführungsform. Die neunte Ausführungsform entspricht an ihrer distalseitigen Begrenzung der dritten Ausführungsform, siehe Fig. 12. Es ist zu erkennen, dass die Neutralelektrode 32 nicht als Draht ausgeführt sein muss, sondern auch flächig ausgeführt sein kann.

Damit wurde insgesamt ein bipolares medizinisches Instrument 10 aufgezeigt, mit dem besonders atraumatisch gearbeitet werden kann und welches Vorteile bei der Herstellung realisiert.

## Patentansprüche

1. Bipolares medizinisches Instrument (10) zum Schneiden von Gewebe unter der Wirkung von Hochfrequenzstrom, wobei sich das Instrument (10) entlang einer Längsrichtung (12) von einem proximalen Ende (14) zu einem distalen Ende (16) erstreckt und wobei am distalen Ende (16) eine Aktivelektrode (30) und eine der Aktivelektrode (30) benachbarten Neutralelektrode (32) angeordnet sind, wobei die Neutralelektrode (32) einen gekrümmten Verlauf mit einer ersten und einer zweiten Krümmung (50, 52) hat und die jeweiligen Richtungen der Krümmungen (50, 52) unterschiedlich sind, **dadurch gekennzeichnet, dass** die Neutralelektrode (32) in der Draufsicht auf eine von der Längsrichtung (12) und einer Querrichtung (24) aufgespannte Ebene entlang der Querrichtung (24) eine W-Form aufweist und eine distalseitige Begrenzung der Neutralelektrode (32) so geformt ist, dass zwischen einem ersten und einem zweiten Bereich (90, 92) der Neutralelektrode (32), die sich distal am weitesten erstrecken, ein dritter Bereich (94) angeordnet ist, der sich distal weniger weit erstreckt als der erste und der zweite Bereich (90, 92) und eine proximalseitige Begrenzung der Neutralelektrode (32) bildet.

2. Instrument nach Anspruch 1, wobei der gekrümmte Verlauf zudem eine dritte Krümmung (54) hat und sich entlang des Verlaufs der Neutralelektrode (32) die Richtungen der Krümmungen (50, 52, 54) zweimal ändern.

3. Instrument nach einem der vorhergehenden Ansprüche, wobei die Neutralelektrode (32) als Drahtelement ausgebildet ist.

4. Instrument nach einem der vorhergehenden Ansprüche, wobei eine erste Oberfläche der Aktivelektrode (30) kleiner ist als eine zweite Oberfläche der Neutralelektrode (32).

5. Instrument nach einem der vorhergehenden Ansprüche, wobei ein Querschnittsprofil der Neutralelektrode (32) eine gerundete Form hat, insbesondere eine zumindest im Wesentlichen ovale oder zumindest im Wesentlichen kreisrunde Form.

6. Instrument nach einem der vorhergehenden Ansprüche, wobei die Neutralelektrode (32) entlang ihres physikalischen Verlaufs einen gleichbleibenden Querschnitt aufweist, insbesondere einen konstanten Durchmesser.

7. Instrument nach einem der vorhergehenden Ansprüche, wobei die Neutralelektrode zumindest zwei gebogene Abschnitte (80. 82) aufweist, insbesondere zumindest drei gebogene Abschnitte (80, 82, 84), die insbesondere jeweils zumindest in etwa die Form eines Teilovals oder eines Teilkreises haben.

8. Instrument nach einem der vorhergehenden Ansprüche, wobei sich die Aktivelektrode (30) und die Neutralelektrode (32) in einem eingefahrenen Zustand innerhalb eines distalen Endes eines Schafts (18) des Instruments (10) befinden.

9. Instrument nach einem der vorhergehenden Ansprüche, wobei, bezogen auf die Längsrichtung (12), ein distales Ende (70) der Aktivelektrode (30) weiter distal angeordnet ist als ein distales Ende (77) der Neutralelektrode (32) oder, bezogen auf die Längsrichtung (12), ein distales Ende (70) der Aktivelektrode (30) distal gleich weit angeordnet ist wie ein distales Ende (72) der Neutralelektrode (32).

10. Instrument nach einem der vorhergehenden Ansprüche, wobei die Neutralelektrode (32) zusammen mit dem Neutralleiter (40) einstückig aus genau einem Material oder genau einer Materialmischung gefertigt ist.

11. Instrument nach einem der vorhergehenden Ansprüche, wobei sich die Neutralelektrode (32) bezogen auf ihre Quererstreckung in einem mittleren Bereich (86) weniger weit distal erstreckt als in Seitenbereichen (88) der Neutralelektrode (32).

12. Instrument nach einem der vorhergehenden Ansprüche, wobei zumindest ein Abschnitt der Neutralelektrode in einer Ebene (60) verläuft, die in einem Winkel zu einer Längserstreckung (12) des Instruments (10) steht.

13. Elektrochirurgisches System mit einem Hochfrequenzgenerator (26) und mit einem Instrument (10) nach einem der Ansprüche 1 bis 12, das an den Hochfrequenzgenerator (26) anschließbar ist.

## Claims

1. A bipolar medical instrument (10) for cutting tissue under the action of high frequency current, wherein the instrument (10) extends along a longitudinal direction (12) from a proximal end (14) to a distal end (16) and wherein an active electrode (30) and a neutral electrode (32) adjacent the active electrode (30) are disposed at the distal end (16), the neutral electrode (32) having a curved shape with a first and a second curvature (50, 52) and the respective directions of the curvatures (50, 52) being different, **characterized in that** the neutral electrode (32) has, in a plan view of a plane spanned by the longitudinal direction (12) and a transverse direction (24) viewed along the transverse direction (24), a W-shape and a distal-sided boundary of the neutral electrode (32) is formed in such a way that between a first and a second region (90, 92) of the neutral electrode (32), which extend distally furthest, a third region (94) extending distally less far than the first and second regions (90, 92) and forming a proximal-side boundary of the neutral electrode (32) is disposed.

2. Instrument according to claim 1, wherein the curved shape further comprises a third curvature (54) and along the shape of the neutral electrode (32) the directions of the curvatures (50, 52, 54) change twice.

3. Instrument according to one of the preceding claims, wherein the neutral electrode (32) is formed as a wire element.

4. Instrument according to one of the preceding claims, wherein a first surface of the active electrode (30) is smaller than a second surface of the neutral electrode (32).

5. Instrument according to one of the preceding claims, wherein a cross-sectional profile of the neutral electrode (32) has a rounded shape, in particular an at least substantially oval or at least substantially circular shape.

6. Instrument according to one of the preceding claims, the neutral electrode (32) having a constant cross-section, in particular a constant diameter, along its physical shape.

7. Instrument according to one of the preceding claims, wherein the neutral electrode has at least two curved portions (80, 82), in particular at least three curved portions (80, 82, 84), which in particular each have at least approximately the shape of a partial oval or a partial circle.

8. Instrument according to one of the preceding claims, the active electrode (30) and the neutral electrode (32), if in a retracted state, are arranged inside a distal end of a shaft (18) of the instrument (10).

9. Instrument according to one of the preceding claims, wherein, with respect to the longitudinal direction (12), a distal end (70) of the active electrode (30) is arranged further distally than a distal end (77) of the neutral electrode (32) or, with respect to the longitudinal direction (12), a distal end (70) of the active electrode (30) is arranged distally as far as a distal end (72) of the neutral electrode (32).

10. Instrument according to one of the preceding claims, wherein the neutral electrode (32) together with the neutral conductor (40) is integrally made of exactly one material or exactly one material mixture.

11. Instrument according to one of the preceding claims, wherein the neutral electrode (32) extends less distally with respect to its transverse extension in a central region (86) than in side regions (88) of the neutral electrode (32).

12. Instrument according to one of the preceding claims, wherein at least a portion of the neutral electrode extends in a plane (60) which is at an angle to a longitudinal extension (12) of the instrument (10).

13. An electrosurgical system comprising a radio frequency generator (26) and an instrument (10) according to any one of claims 1 to 12 connectable to the radio frequency generator (26).

## Revendications

1. Instrument médical bipolaire (10) pour couper du tissu sous l'action d'un courant à haute fréquence, dans lequel l'instrument (10) s'étend le long d'une direction longitudinale (12) d'une extrémité proximale (14) à une extrémité distale (16) et dans lequel une électrode active (30) et une électrode neutre (32) adjacente à l'électrode active (30) sont disposées à l'extrémité distale (16), l'électrode neutre (32) ayant une trajectoire incurvée avec une première et une seconde courbure (50, 52) et les directions respectives des courbures (50, 52) étant différentes, **caractérisée en ce que** l'électrode neutre (32) dans la vue en plan d'un plan traversé par la direction longitudinale (12) et une direction transversale (24) le long de la direction transversale (24) a une forme en W, et une limite distale de l'électrode neutre (32) est formée de telle manière qu'entre une première et une seconde région (90, 92) de l'électrode neutre (32), qui s'étendent le plus distalement, une troisième région (94) est disposée qui s'étendant distalement moins loin que la première région et la deuxième région (90, 92) et forme une limite côté proximal de l'électrode neutre (32).

2. Instrument selon la revendication 1, dans lequel la trajectoire incurvé comprend en outre une troisième courbure (54) et le long de la trajectoire de l'électrode neutre (32), les directions des courbure (50, 52, 54) changent deux fois.

3. Instrument selon l'une des revendications précédentes, dans lequel l'électrode neutre (32) est réalisée sous la forme d'un élément filaire.

4. Instrument selon l'une des revendications précédentes, dans lequel une première surface de l'électrode active (30) est plus petite qu'une seconde surface de l'électrode neutre (32).

5. Instrument selon l'une des revendications précédentes, dans lequel un profil en coupe transversale de l'électrode neutre (32) présente une forme arrondie, en particulier une forme au moins sensiblement ovale ou au moins sensiblement circulaire.

6. Instrument selon l'une des revendications précédentes, dans lequel l'électrode neutre (32) a une section transversale constante, en particulier un diamètre constant, le long de son trajectoire physique.

7. Instrument selon l'une des revendications précédentes, dans lequel l'électrode neutre présente au moins deux parties courbes (80, 82), en particulier au moins trois parties courbes (80, 82, 84), qui en particulier présentent chacune au moins approximativement la forme d'un ovale partiel ou d'un cercle partiel.

8. Instrument selon l'une des revendications précédentes, dans lequel l'électrode active (30) et l'électrode neutre (32) sont à l'état rétracté dans une extrémité distale d'une tige (18) de l'instrument (10).

9. Instrument selon l'une des revendications précédentes, dans lequel, par rapport à la direction longitudinale (12), une extrémité distale (70) de l'électrode active (30) est disposée plus distalement qu'une extrémité distale (77) de l'électrode neutre (32) ou, par rapport à la direction longitudinale (12), une extrémité distale (70) de l'électrode active (30) est disposée aussi loin que distal (72) de l'électrode neutre (32).

10. Instrument selon l'une des revendications précédentes, dans lequel l'électrode neutre (32) et le conducteur neutre (40) sont réalisés d'un seul tenant avec exactement un matériau ou exactement un mélange de matériaux.

11. Instrument selon l'une des revendications précédentes, dans lequel l'électrode neutre (32) s'étend moins distalement par rapport à son extension transversale dans une zone centrale (86) que dans des zones latérales (88) de l'électrode neutre (32).

12. Instrument selon l'une des revendications précédentes, dans lequel au moins une partie de l'électrode neutre s'étend dans un plan (60) qui est en angle avec une extension longitudinale (12) de l'instrument (10).

13. Système électrochirurgical comprenant un générateur de radiofréquence (26) et un instrument (10) selon l'une quelconque des revendications 1 à 12 pouvant être connecté au générateur de radiofréquence (26).
